# EUROPEAN PATENT SPECIFICATION

(11) **EP 4 241 756 B1**
(45) Date of publication and mention of the grant of the patent: **16.10.2024**
(21) Application number: 22182802.3
(22) Date of filing: 04.07.2022
(51) Int. Cl.: A61K 8/02, A61K 8/73, A61Q 19/08

(54) **ANTI-WRINKLE COMPOSITION, ANTI-WRINKLE MICRONEEDLE PATCH AND PREPARATION METHOD THEREOF**
ANTI-FALTEN-ZUSAMMENSETZUNG, ANTI-FALTEN-MIKRONADELPFLASTER UND HERSTELLUNGSVERFAHREN DAVON
COMPOSITION ANTI-RIDES, TIMBRE À MICRO-AIGUILLE ANTI-RIDES ET SON PROCÉDÉ DE PRÉPARATION

(30) Priority: 07.03.2022 TW 111108251
(43) Date of publication of application: 13.09.2023
(73) Proprietor: WCC BIOMEDICAL CO., LTD., Hsinchu City (TW)
(72) Inventor: LIU, Ta-Jo, Hsinchu City (TW); YEH, Hsiu-Feng, Hsinchu City (TW); HSU, Ying-Hua, Hsinchu City (TW)
(74) Representative: Becker Kurig & Partner Patentanwälte mbB

(56) References cited:
- US-A1- 2015 118 322
- US-A1- 2018 236 215
- DATABASE GNPD [online] MINTEL; 23 March 2020 (2020-03-23), ANONYMOUS: "Hyaluronic Acid Microneedle Filler Patch", XP093044358, retrieved from https://www.gnpd.com/sinatra/recordpage/7448011/ Database accession no. 7448011

## Description

The present invention relates to a composition, a microneedle patch and a preparation method thereof, and more particularly to an anti-wrinkle composition, an anti-wrinkle microneedle patch, and a preparation method thereof.

Recently, transdermal drug delivery has become a high-profile non-invasive way of drug administration that exerts the effects of active ingredients, such as drugs or vaccines, by skin absorption. Although the transdermal drug delivery can avoid drug degradation caused by the digestive system and primary liver metabolism resulting from oral administration and also can eliminate the fear and pain resulting from subcutaneous injection, the conventional transdermal drug delivery is not suitable for water-soluble drugs or water-soluble vaccines due to the hydrophobic and negatively charged properties of the stratum corneum of skin.

For the aforementioned drawbacks, the prior art has developed a microneedle patch with multiple micron-sized microneedle structures on its substrate. Those microneedle structures can pierce the stratum corneum of the skin, and deliver and release drugs or vaccines to the epidermis. Drug administration with the microneedle patch not only solves many problems caused by oral administration or subcutaneous injection, but also further expands the types of delivered drugs or vaccines to fat-soluble and water-soluble, such that the foresaid different kinds of drugs or vaccines can be directly delivered and released to the epidermis or the dermis by the microneedle structures of the microneedle patch without causing any pain.

Based on the advantages of the microneedle patch, in addition to pharmaceutical and vaccine fields, the microneedle patch is also gradually applied to aesthetic medicine related fields, such as skin cosmetic care.

Hyaluronic acid (HA) is highly hydrophilic and water-retaining, and is widely present in cartilage tissue, body fluids or epidermal tissue of the human body to provide elasticity and lubrication. Therefore, HA has become a well-known skin cosmetic care product, and has effects of skin moisturizing and wrinkle reduction, etc. At present, most of the HA-containing skin cosmetic care products still achieve the purpose of supplementing HA by direct application to the skin, oral drinks and tablets, or subcutaneous injection. However, as mentioned above, both directly applying to the skin and oral administration cannot have good absorption effect, and subcutaneous injection has limitations, such as causing pain and difficulty of operating. Besides, although HA can be made into a microneedle patch for administration, the strength of the needle thereof is usually insufficient due to the property of HA, such that the needle is easily bent and broken when penetrating the skin, which cannot actually deliver and release HA to the epidermis or the dermis.

For the foresaid problem, CN 107207784 A discloses a HA-containing microneedle patch that adopts cross-linked HA as a major material of the needle to enhance the structural strength, thereby reducing the bending of the needle when penetrating the skin. Nevertheless, after the microneedle patch penetrates the skin, the dissolution rate of releasing HA is closely related to the skin cosmetic effects, such as skin moisturizing and wrinkle reduction, etc., while the HA-containing microneedle patch disclosed by CN 107207784 A does not have high dissolution rate of releasing HA. US2018/236215 discloses a microneedle patch and US2015/118322 discloses hyaluronic acid modified with histidine

Therefore, there is still a need to develop and research on a HA-containing microneedle patch whose needle has good structural strength for penetrating the skin and simultaneously has high dissolution rate of releasing HA, such that skin cosmetic effects such as skin moisturization and wrinkle reduction can be accelerated.

Based on the above-mentioned drawbacks of the prior arts, the objective of the present invention is to provide an anti-wrinkle composition, and the anti-wrinkle composition can be the material of the needle to further produce an anti-wrinkle microneedle patch, whose needle has mechanical strength capable of penetrating the skin and simultaneously has high dissolution rate of releasing HA, thereby exerting the effects of skin moisturizing, increasing skin thickness and strength, and smoothing and reducing wrinkles.

To achieve the above-mentioned objective, the present invention provides an anti-wrinkle composition, which comprises a low-molecular HA, a modified HA and a cross-linked HA, and a weight ratio of the modified HA relative to the low-molecular HA is more than or equal to 0.1 and less than or equal to 4, and a weight ratio of the modified HA relative to the cross-linked HA is less than 30; wherein, an average molecular weight of the low-molecular HA is more than or equal to 1 kilodalton (kDa) and less than or equal to 10 kDa, and the modified HA indicates that HA is modified with histidine.

By simultaneously adopting the specific kind of HA and controlling their contents relative to each other, the anti-wrinkle composition of the present invention is used as the material of the needle of a microneedle patch, which makes the needle have good mechanical strength for penetrating the skin, and also makes HA rapidly dissolve and released, so as to exert the effects of skin moisturizing and increasing skin thickness and strength, and achieve the purpose of smoothing and reducing wrinkles.

After the low-molecular HA dissolves in water with a content of 1 weight percent (wt%), a pH value is 5.0 to 7.0, and a viscosity is less than 2 mPa.s (cP) when measured at a shear rate of 1 s⁻¹ at 25°C. Specifically, said "HA modified with histidine" indicates that histidine is linked to hydroxyl group of HA. More specifically, the modified HA may comprise HA modified with histidine, HA and histidine, and said HA and said histidine are unreacted matters after modification.

Preferably, after the modified HA dissolves in water with a content of 1 wt%, a pH value is 5.0 to 7.0, and a viscosity is 2 mPa.s (cP) to 9 mPa.s (cP) when measured at a shear rate of 1 s⁻¹ at 25°C.

In accordance with the present invention, the cross-linked HA is prepared by subjecting HA to a cross-linking agent for cross-linking reaction. Specifically, the cross-linking agent may be 1,4-butanediol diglycidyl ether (BDDE), divinyl sulfone (DVS), adipic dihydrazide (ADH), 1-ehtyl-3-(3-dimethylaminopropyl)carbodiimide (EDC) or glycidyl methacylate (GMA).

Preferably, after the cross-linked HA dissolves in water with a content of 1 wt% to 5 wt%, a pH value is 5.0 to 7.5, and a viscosity is 4000 mPa.s (cP) to 8000 mPa.s (cP) when measured at a shear rate of 1 s⁻¹ at 25°C.

Preferably, the anti-wrinkle composition may further comprise an antiseptic. Specifically, the antiseptic may be, but is not limited to,*p*-hydroxyacetophenone, pentylene glycol, hexanediol, caprylyl glycol, phenoxyethanol, benzyl alcohol, salicylic acid, benzoic acid, sorbic acid, potassium sorbate, propionic acid, dehydroacetic acid, chlorophenesin, behentrimonium chloride, benzalkonium chloride, benzethonium chloride, butyl benzoate, dimethyl oxazolidine, bromochlorophene, dichlorobenzyl alcohol, ethyl lauroyl arginate HCl, 7-ethylbicyclooxazolidine, formic acid, glutaral, hexamidine, sodium sulfite, iodopropynyl butylcarbamate, methylisothiazolinone, butylparaben, methylparaben, phenoxyisopropanol, phenylmercuric acetate, triclocarban, undecylenic acid, zinc pyrithione, 5-bromo-5-nitro-1,3-dioxane, benzylhemiformal, 1,3-dimethylol-5,5-dimethylhydantoin (DMDM Hydantoin), diazolidinyl urea, imidazolidinyl urea, methenamine, quaternium-15, sodium hydroxymethylglycinate, Leuconostoc/Radish Root Ferment Filtrate, Lactobacillus ferment, Populus Tremuloides Bark Extract, Black Currant Fruit Extract or any combination thereof. More preferably, the antiseptic comprises p-hydroxyacetophenone, pentylene glycol, Lactobacillus ferment or any combination thereof.

In some embodiments of the present invention, the anti-wrinkle composition may further comprise a surfactant. Specifically, the surfactant may comprise, but is not limited to, polysorbate 20 (Tween-20), arachidyl glucoside, decyl glucoside, lauryl glucoside, C12-20 alkyl glucoside, PEG-6 caprylic/capric glycerides, polyglyceryl-3 caprate, polyglyceryl-4 stearate, polyglyceryl-10 distearate, polyglyceryl-10 laurate, polyglyceryl-10 isostearate, PEG-7 glyceryl cocoate, polyethylene glycol, sorbitan olivate, sorbitan palmitate or any combination thereof.

In some embodiments of the present invention, the anti-wrinkle composition comprises p-hydroxyacetophenone and pentylene glycol, and based on a total weight of the anti-wrinkle composition, a content of *p*-hydroxyacetophenone is more than or equal to 0.05 wt% and less than or equal to 1 wt%, and a content of pentylene glycol is more than or equal to 0.05 volume-in-weight percent (v/w%) and less than or equal to 1 v/w%. Preferably, based on the total weight of the anti-wrinkle composition, the content of *p*-hydroxyacetophenone is more than or equal to 0.05 wt% and less than or equal to 0.5 wt%, and the content of pentylene glycol is more than or equal to 0.05 v/w% and less than or equal to 0.5 v/w%.

In some embodiments of the present invention, without affecting the effects of the anti-wrinkle composition of the present invention, the anti-wrinkle composition may further comprise, but is not limited to, a whitening ingredient, a moisturizing ingredient, an antioxidant, an anti-wrinkle ingredient or any combination thereof. For example, the whitening ingredient may be, but is not limited to, kojic acid, arbutin, sodium ascorbyl phosphate, magnesium ascorbyl phosphate, ascorbyl glycoside, ellagic acid, chamomile ET, cetyltranexamate HCl, potassium 4-methoxysalicylate, 3-O-ethyl ascorbic acid or nonapeptide; the moisturizing ingredient may be, but is not limited to, ceramide, lecithin, glycerol, polysaccharide, protein, collagen, elastin, peptide, amino acid, citrate, uric acid, urea, glucose, sucrose, fructose, glycogen, glucosamine, mucopolysaccharides, lactate, phosphate or ethyl-dl-2-pyrrolidone-5-carboxylate; the antioxidant may be, but is not limited to, grape extract, green tea extract, ginkgo extract, soy extract, pomegranate extract, ginger extract, yeast extract, coix extract, R-alpha-lipoic acid, glucan, coenzyme Q10, superoxide dismutase (SOD), vitamin C and the derivatives thereof or vitamin E and the derivatives thereof; and the anti-wrinkle ingredient may be, but is not limited to, retinol and the derivatives thereof, copper peptide (GHK-Cu), pentapeptide or hexapeptide.

Preferably, the weight ratio of the modified HA relative to the low-molecular HA is more than or equal to 0.1 and less than or equal to 3. More preferably, the weight ratio of the modified HA relative to the low-molecular HA is more than or equal to 0.1 and less than or equal to 2.

Preferably, the weight ratio of the modified HA relative to the cross-linked HA is more than or equal to 2 and less than or equal to 25. More preferably, the weight ratio of the modified HA relative to the cross-linked HA is more than or equal to 2 and less than or equal to 23.

Besides, to achieve the above-mentioned objective, the present invention further provides an anti-wrinkle microneedle patch, which comprises a base and multiple needles; the base has an upper surface and the multiple needles are formed on the upper surface; wherein, a material of each needle comprises the anti-wrinkle composition mentioned above.

By adopting the anti-wrinkle composition having specific composition as the material of the needle of the anti-wrinkle microneedle patch, the needles have good mechanical strength for penetrating the skin, and also HA can rapidly dissolve and be released to exert the effects of skin moisturizing and increasing skin thickness and strength, and achieve the purpose of smoothing and reducing wrinkles.

In accordance with the present invention, a material of the base may be dissolvable or swellable polymer materials. Specifically, the polymer materials may be biocompatible materials or biodegradable materials. For example, the material of the base may be, but is not limited to, hydroxypropyl methylcellulose (HPMC), polyvinylpyrrolidone/vinyl acetate (PVP/VA) or a combination thereof.

In some embodiments of the present invention, the material of the base comprises HPMC, PVP/VA, Tween-20, an antiseptic containing Lactobacillus ferment, p-hydroxyacetophenone and pentylene glycol, and an organic silicon defoamer.

In accordance with the present invention, a mechanical strength of the needle of the anti-wrinkle microneedle patch is more than 0.058 N/needle, such that the anti-wrinkle microneedle patch of the present invention is able to pierce the stratum corneum without breakage. Preferably, the mechanical strength of the needle of the anti-wrinkle microneedle patch is more than 0.08 N/needle. More preferably, the mechanical strength of the needle of the anti-wrinkle microneedle patch is more than 0.1 N/needle.

In accordance with the present invention, a dissolution rate in water for 5 minutes of the needle of the anti-wrinkle microneedle patch is more than 60%. Preferably, the dissolution rate in water for 5 minutes of the needle of the anti-wrinkle microneedle patch is more than or equal to 65% and less than or equal to 90%. More preferably, the dissolution rate in water for 5 minutes of the needle of the anti-wrinkle microneedle patch is more than or equal to 75% and less than or equal to 90%. Even more preferably, the dissolution rate in water for 5 minutes of the needle of the anti-wrinkle microneedle patch is more than or equal to 80% and less than or equal to 90%.

In some embodiments of the present invention, a thickness of the needle, *i.e.,* a needle length of the needle, is more than or equal to 270 micrometers (µm) and less than or equal to 330 µm.

In some embodiments of the present invention, a thickness of the base is more than or equal to 40 µm and less than or equal to 90 µm.

In accordance with the present invention, a needle shape of each needle may be, but is not limited to, conical, pyramid-shaped or steeple-shaped.

In accordance with the present invention, a density of the needles may range from 1 needle/cm² to 1000 needles/cm²; preferably, range from 1 needle/cm² to 500 needles/cm².

Besides, the present invention further provides a preparation method of the foresaid anti-wrinkle microneedle patch, which comprises the following steps:
step (a): providing a master mold having a datum plane and multiple holes, and the multiple holes formed by recessing from the datum plane;
step (b): filling the multiple holes with a needle solution to make a surface of the needle solution level with the datum plane; wherein the needle solution comprises a low-molecular HA, a modified HA and a cross-linked HA, and a weight ratio of the modified HA relative to the low-molecular HA is more than or equal to 0.1 and less than or equal to 4, and a weight ratio of the modified HA relative to the cross-linked HA is less than 30; wherein, an average molecular weight of the low-molecular hyaluronic acid is more than or equal to 1 kDa and less than or equal to 10 kDa, and the modified HA indicates that HA is modified with histidine;
step (c): drying the needle solution to form multiple needles, and a surface of each needle is lower than the datum plane;
step (d): filling the multiple holes with a base solution, and the base solution covers the surface of the needles and the datum plane;
step (e): drying the base solution to form a base, such that the base is adhered to the multiple needles; and
step (f): demolding the needles and the base that are adhered to each other from the master mold to obtain the anti-wrinkle microneedle patch.

According to the preparation method of the present invention, the anti-wrinkle microneedle patch made therefrom has good mechanical strength capable of penetrating the skin, and also makes HA rapidly dissolve and released, so as to exert the effects of skin moisturizing and increasing skin thickness and strength, and achieve the purpose of smoothing and reducing wrinkles.

Preferably, a sum of a weight of the low-molecular HA, a weight of the modified HA, and a weight of the cross-linked HA in 1000 grams (g) of the needle solution is more than or equal to 20 g and less than or equal to 70 g. More preferably, the sum of the weight of the low-molecular HA, the weight of the modified HA, and the weight of the cross-linked HA in 1000 g of the needle solution is more than or equal to 25 g and less than or equal to 60 g.

Preferably, a viscosity of the needle solution is measured at a shear rate of 1 s⁻¹ at 25°C, and the viscosity of the needle solution is more than or equal to 2 mPa.s (cP) and less than or equal to 50 mPa.s (cP). More preferably, the viscosity of the needle solution is measured at a shear rate of 1 s⁻¹ at 25°C, and the viscosity of the needle solution is more than or equal to 3 mPa.s (cP) and less than or equal to 30 mPa.s (cP).

In some embodiments of the present invention, the needle solution is prepared by uniformly mixing the foresaid anti-wrinkle composition and water.

In some embodiments of the present invention, the needle solution is prepared by uniformly mixing the low-molecular HA, the modified HA, a cross-linked HA aqueous solution and water; wherein, a solid content of the cross-linked HA aqueous solution is 4.4 wt% to 4.6 wt%, and a viscosity of the cross-linked HA aqueous solution is more than or equal to 4000 mPa.s (cP) and less than or equal to 8000 mPa.s (cP) when measured at a shear rate of 1 s⁻¹ at 25°C.

In some embodiments of the present invention, the step (b) may further comprise: step (b 1): forming the needle solution on the master mold, and making the needle solution flow into the multiple holes to cover the datum plane and the multiple holes; and step (b2): removing the needle solution above the datum plane to make the surface of the needle solution level with the datum plane.

Preferably, a way of filling the needle solution into the multiple holes may comprise vacuum evacuation and centrifugation in the step (b). In some embodiments of the present invention, the needle solution and the master mold may be placed in an oven for evacuation to make the needle solution cover the datum plane and the multiple holes; in another embodiments of the present invention, the needle solution and the master mold may be centrifuged together to make the needle solution cover the datum plane and the multiple holes. Here, a pressure of the oven may be -700 mmHg to -760 mmHg, and a rotational speed of the centrifugation may be 20 xg to 20000 xg; preferably, 20 xg to 12000 xg. More preferably, the way of filling the needle solution into the multiple holes is vacuum evacuation in the step (b).

Preferably, a way of filling the base solution into the multiple holes may comprise vacuum evacuation and centrifugation in the step (d). In some embodiments of the present invention, the base solution and the master mold may be placed in an oven for evacuation to make the base solution cover the datum plane and the multiple holes; in another embodiments of the present invention, the base solution and the master mold may be centrifuged together to make the base solution cover the datum plane and the multiple holes. Here, a pressure of the oven may be - 700 mmHg to -760 mmHg, and a rotational speed of the centrifugation may be 20 xg to 20000 xg; preferably, 20 xg to 12000 xg. More preferably, the way of filling the base solution into the multiple holes is vacuum evacuation in the step (d).

In some embodiments of the present invention, the master mold may be hard master mold, and materials of the hard master mold may be, but are not limited to, glass, quartz, silicon wafer, metal, metal oxide or metal alloy. The said metal may be, but is not limited to, aluminum, copper or nickel. In another embodiments of the present invention, the master mold may be soft master mold, and materials of the soft master mold may be, but are not limited to, polymer, metal foil or flexible glass. The said polymer may be, but is not limited to, poly(dimethylsiloxane) (PDMS), poly(methylmethacrylate) (PMMA), polycarbonate (PC) or polyethersulfone (PES).

Preferably, in the step (b) and step (d), respectively, the needle solution and the base solution may be formed on the master mold by, but not limited to, slit or slot die coating, blade coating, slide coating, dip coating, inkjet printing, nozzle printing, dispenser or any combination thereof. The needle solution in the step (b) and the base solution in the step (d) may be formed on the master mold by the same way or different ways. Preferably, in the preparation method of the present invention, the needle solution and the base solution may be sequentially formed on the master mold by slit or slot die coating. More preferably, in the preparation method of the present invention, the needle solution and the base solution may be sequentially formed on the master mold by a dispenser.

In some embodiments of the present invention, when slit or slot die coating is adopted to coat the needle solution in the step (b), the coating gap may be 1 µm to 5000 µm, and the coating speed may be 1 meter/minute (m/min) to 100 m/min. The process parameters are adjustable depending on the properties of the needle solution and the specification of the microneedle patch. When slit or slot die coating is adopted to coat the base solution in the step (d), the coating gap may be 1 µm to 3000 µm, and the coating speed may be 1 m/min to 100 m/min. The process parameters are adjustable depending on the properties of the base solution and the specification of the microneedle patch.

Preferably, in the step (b), the coating gap may be 100 µm to 5000 µm, and the coating speed may be 1 m/min to 100 m/min; in the step (d), the coating gap may be 100 µm to 3000 µm, and the coating speed may be 1 m/min to 100 m/min.

Preferably, the step (c) may be accomplished by drying at room temperature or heating. More preferably, the step (c) is accomplished by drying at room temperature, and the drying temperature is 20°C to 35°C, the ambient relative humidity (RH) is 10% to 40%, and the time is 0.5 hour (hr) to 3 hr.

Preferably, the step (e) may be accomplished by drying at room temperature or heating. More preferably, the step (e) is accomplished by drying at room temperature, and the drying temperature is 20°C to 35°C, the ambient RH is 50% to 80%, and the time is 20 hr to 72 hr.

In some embodiments of the present invention, the step (e) is accomplished by two-step drying. Specifically, in the first drying step, the drying temperature is 20°C to 35°C, the ambient RH is 30% to 50%, and the time is 10 hr to 24 hr; and in the second drying step, the drying temperature is 20°C to 35°C, the ambient RH is 50% to 80%, and the time is 10 hr to 72 hr.

In accordance with the present invention, the shape of the multiple holes of the master mold may be, but is not limited to, conical, pyramid-shaped or steeple-shaped. The master mold has a datum plane and multiple holes, and each hole is formed by recessing from the datum plane. The depth of each hole ranges from 75 µm to 1500 µm; preferably, from 150 µm to 1200 µm; more preferably, from 175 µm to 1000 µm; even more preferably, from 200 µm to 1000 µm; and even more preferably from 270 µm to 900 µm. The maximum width of each hole ranges from 38 µm to 800 µm; preferably, from 75 µm to 650 µm; and more preferably, from 85 µm to 550 µm.

In the specification, said "hyaluronic acid" indicates hyaluronic acid, hyaluronate or a combination thereof; wherein, said "hyaluronate" may comprise sodium hyaluronate, potassium hyaluronate, magnesium hyaluronate or calcium hyaluronate.

In the specification, a range represented by "a lower-endpoint value to an upper-endpoint value", if not particularly specified, indicates that the range is more than or equal to the lower-endpoint value and less than or equal to the upper-endpoint value. For example, "a density of the needles may range from 1 needle/cm² to 1000 needles/cm²" indicates that the density of the needles is "more than or equal to 1 needle/cm² and less than or equal to 1000 needles/cm²".

Other objectives, advantages and novel features of the invention will become more apparent from the following detailed description.

Several preparation methods of embodiments are exemplified below to illustrate the implementation of the present invention. One person skilled in the art can easily realize the advantages and effects of the present invention in accordance with the contents disclosed in the specification.

### Description of Reagents

1. Low-molecular HA: purchased from Kewpie corporation; the average molecular weight is about 2 kDa; after dissolving in water with 1 wt%, the pH value is 5.0 to 7.0, and the viscosity is less than 2 mPa.s (cP) when measured at a shear rate of 1 s⁻¹ at 25°C.
2. Histidine-modified HA: purchased from Industrial Technology Research Institute; after dissolving in water with 1 wt%, the pH value is 5.0 to 7.0, and the viscosity is about 4 mPa.s (cP) to 5 mPa.s (cP) when measured at a shear rate of 1 s⁻¹ at 25°C.
3. Cross-linked HA aqueous solution 1: purchased from Industrial Technology Research Institute; BDDE is used as the cross-linking agent; the solid content is about 4.5 wt%; the pH value is 5.0 to 7.5; and the viscosity is about 5000 mPa.s (cP) to 8000 mPa.s (cP) when measured at a shear rate of 1 s⁻¹ at 25°C.
4. Cross-linked HA aqueous solution 2: purchased from Bloomage Biotechnology Corporation Limited.; BDDE is used as the cross-linking agent; the solid content is about 5 wt%; the pH value is 5.0 to 7.5; and the viscosity is more than or equal to 250000 mPa.s (cP) when measured at a shear rate of 1 s⁻¹ at 25°C.
5. Hyaluronic acid: purchased from Kewpie corporation; the average molecular weight is less than or equal to 10 kDa; after dissolving in water with 0.1 wt%, the pH value is 2.0 to 4.0, and the viscosity is less than 1.5 mPa.s (cP) when measured at a shear rate of 1 s⁻¹ at 25°C.
6. Carboxymethyl cellulose (CMC): purchased from Shin-Etsu Chemical Co., Ltd.; the average molecular weight is about 90 kDa; after dissolving in water with 4 wt%, the pH value is 6.5 to 8.5, and the viscosity is more than or equal to 50 mPa.s (cP) and less than or equal to 200 mPa.s (cP) when measured at a shear rate of 1 s⁻¹ at 25°C.
7. p-Hydroxyacetophenone: purchased from Symrise corporation.
8. Pentylene glycol: purchased from ACTIVON Corporation.
9. Polysorbate 20 (Tween-20): purchased from CRODA Corporation.
10. First HPMC: purchased from Shin-Etsu Chemical Co., Ltd.; product name: 60SH-4000; after dissolving in water with 2 wt%, the viscosity is about 3000 mPa.s (cP) to 5000 mPa.s (cP) when measured at a shear rate of 1 s⁻¹ at 20°C.
11. Second HPMC: purchased from Shin-Etsu Chemical Co., Ltd.; product name: PHARMACOAT 645; after dissolving in water with 2 wt%, the viscosity is about 3.6 mPa.s (cP) to 5.1 mPa.s (cP) when measured at a shear rate of 1 s⁻¹ at 20°C.
12. Polyvinylpyrrolidone/vinyl acetate (PVP/VA): purchased from BASF Corporation.
13. Lactobacillus ferment: purchased from ACTIVE MICRO Corporation; product name: Leucidal^{®} SF MAX.
14. Organic silicon defoamer: purchased from Dow Corning Corporation; product name: DOWSIL^{™} 62 Additive.

### Examples 1 to 8: anti-wrinkle microneedle patch

First, a master mold with a datum plane and multiple holes was adopted. The multiple holes were formed by recessing from the datum plane, and arranged in array on the master mold. The material of the master mold was polydimethylsiloxane (PDMS), the density of the multiple holes was 75.08 holes/cm², the multiple holes were arranged in a square array with a side length of 1 cm, and each hole was in the shape of a pyramid. The depth of each hole, *i.e.* the vertical distance between the tip of each hole and the datum plan, was about 270 µm to 330 µm, and the maximum width of each hole, *i.e.* the maximum inner diameter of the cross-section of each hole that aligned to the datum plane, was about 130 µm to 170 µm.

Next, according to the compositions listed in the following Table 1, suitable amounts of the low-molecular HA, the histidine-modified HA, the cross-linked HA aqueous solution 1, p-hydroxyacetophenone and pentylene glycol were added into water and mixed uniformly to obtain a needle solution. Afterward, about 5 g to 7 g of the needle solution was dropped on the master mold and covered the multiple holes thereof by a dispenser. Then, the master mold with the needle solution was placed into a vacuum oven for evacuation to reduce the pressure to -700 mmHg to -760 mmHg, such that the needle solution flowed downward into the multiple holes from the datum plane, and covered the datum plane and all holes. Next, the needle solution above the datum plane was totally removed to make the surface of the needle solution level with the datum plane, and the master mold with the needle solution was placed into an environment at 30°C and with the RH of 10% to 30% for 1 hr to dry the needle solution into multiple needles. The surface of the needles was all lower than the datum plane, and a master mold with multiple needles was obtained. The viscosity of the needle solutions of Examples 1 to 8 was measured by a viscometer (model: MCR302; purchased from Anton Paar) at a shear rate of 1 s⁻¹ at 25°C, and the results were listed in the following Table 1. Besides, the sum of the weight of the low-molecular HA, the weight of the histidine-modified HA and the weight of the cross-linked HA relative to the total weight of 1000 g of the needle solution, abbreviated as "total amount of three HA in needle solution", of Examples 1 to 8 were also listed in the following Table 1, and the unit was g/kg. Said weight of the cross-linked HA indicated the weight of solid, which could be calculated by multiplying the weight of the cross-linked HA aqueous solution 1 to its solid content (4.5 wt%). For instance, in Example 1, the needle solution contained 6.72 wt% of the cross-linked HA aqueous solution 1, which indicated that 1000 g of the needle solution contained 67.2 g of the cross-linked HA aqueous solution 1. Then, the weight of the cross-linked HA aqueous solution 1 was multiplied to its solid content of 4.5 wt% to obtain the weight of the cross-linked HA, *i.e.,* 3.024 g. Therefore, in Example 1, the sum of the weight of the low-molecular HA, the weight of the histidine-modified HA and the weight of the cross-linked HA relative to the total weight of 1000 g of the needle solution was the sum of 1000 g×4.03 wt%, 1000 g×1.34 wt% and 3.024 g, and was equal to 56.72 g. Besides, the weight ratio of the histidine-modified HA relative to the low-molecular HA and the weight ratio of the histidine-modified HA relative to the cross-linked HA were also listed in the following Table 1. It should be understood that the weight of the cross-linked HA was obtained by multiplying the weight of the cross-linked HA aqueous solution 1 to its solid content (4.5 wt%), and the weight ratio of the histidine-modified HA relative to the cross-linked HA can be calculated by the ratio of the weight of the histidine-modified HA relative to the weight of the cross-linked HA. For instance, in Example 1, the needle solution contained 6.72 wt% of the cross-linked HA aqueous solution 1, which indicated that the needle solution contained 0.3024 wt% of the cross-linked HA. Then, the cross-linked HA was further compared to the histidine-modified HA (1.34 wt%) to obtain the weight ratio of the histidine-modified HA relative to the cross-linked HA was about 4.43.

Next, suitable amounts of first HPMC, second HPMC, PVP/VA, p-hydroxyacetophenone, pentylene glycol, Tween-20, Lactobacillus ferment and organic silicon defoamer were added into water and mixed uniformly to obtain a base solution; wherein, based on the total weight of the base solution, the content of the first HPMC was about 0.96 wt%, the content of the second HPMC was about 0.48 wt%, the content of PVP/VA was about 1.92 wt%, the content of Tween-20 was about 0.02 v/w%, the content of the Lactobacillus ferment was about 0.8 wt%, the content of *p*-hydroxyacetophenone was about 0.25 wt%, the content of pentylene glycol was about 0.16 v/w%, and the content of the organic silicon defoamer was about 0.02 wt%. Afterward, about 7 g to 8.5 g of the base solution was dropped on the master mold with multiple needles by a dispenser, and covered the multiple holes thereof. Then, the master mold with the base solution was placed into the vacuum oven for evacuation to reduce the pressure to -700 mmHg to -760 mmHg, such that the base solution flowed downward into the multiple holes from the datum plane, and covered the datum plane and all needles in the holes. Next, the master mold with the base solution was placed into an environment at 23°C and with the RH of 30% to 50% for 24 hr, and subsequently placed into an environment at 23°C and with the RH of 55% to 75% for 60 hr to dry the base solution into the base. The base was adhered to the needles and the datum plane of the master mold, and a master mold with multiple needles and the base was obtained.

Finally, the final product was demolded from the master mold with multiple needles and the base to obtain the anti-wrinkle microneedle patches of Examples 1 to 8.

### Comparative Examples 1 to 3: anti-wrinkle microneedle patch

The methods of preparing the anti-wrinkle microneedle patches of Comparative Examples 1 to 3 were similar to those of Examples. The main difference was that the needle solutions were prepared according to the compositions of Comparative Examples 1 to 3 listed in the following Table 1, and the rest of the preparation was in accordance with the descriptions in Examples to obtain the anti-wrinkle microneedle patches of Comparative Examples 1 to 3. The viscosity of the needle solutions of Comparative Examples 1 to 3 was measured by the viscometer at a shear rate of 1 s⁻¹ at 25°C, and the results were listed in the following Table 1. Besides, the results of total amount of three HA in needle solution of Comparative Examples 1 to 3 were also listed in the following Table 1. Also, the weight ratio of the histidine-modified HA relative to the low-molecular HA and the weight ratio of the histidine-modified HA relative to the cross-linked HA were also listed in the following Table 1.

### Comparative Examples 4 to 7: anti-wrinkle microneedle patch

The methods of preparing the anti-wrinkle microneedle patches of Comparative Examples 4 to 7 were similar to those of Examples. The main difference was that the needle solutions were prepared according to the compositions of Comparative Examples 4 to 7 listed in the following Table 2, and the rest of the preparation was in accordance with the descriptions in Examples to obtain the anti-wrinkle microneedle patches of Comparative Examples 4 to 7. The solid content of the needle solutions and the viscosity of the needle solutions measured by the viscometer at a shear rate of 1 s⁻¹ at 25°C of Comparative Examples 4 to 7 were also listed in the following Table 2.

**Table 1: The composition of the needle solution, the weight ratio of the histidine-modified HA relative to the low-molecular HA, the weight ratio of the histidine-modified HA relative to the cross-linked HA, the total amount of three HA in needle solution, and viscosity of Examples 1 to 8 (E1 to E8) and Comparative Examples 1 to 3 (CE1 to CE3).**

| No. | The composition of the needle solution | | | | | Weight ratio of the histidine-modified HA relative to the low-molecular HA | Weight ratio of the histidine-modified HA relative to the cross-linked HA | Total amount of three HA in needle solution (g/kg) | Viscosity mPa. s (cP) |
|---|---|---|---|---|---|---|---|---|---|
| | Low-molecular HA (wt%) | Histidine-modified HA (wt%) | Cross-linked HA aqueous solution 1 (wt%) | *p-*Hydroxyacetophenone (wt%) | Pentylene glycol (v/w%) | | | | |
| E1 | 4.03 | 1.34 | 6.72 | 0.4 | 0.11 | 0.33 | 4.43 | 56.72 | 10.18 |
| E2 | 4.03 | 1.35 | 1.34 | 0.4 | 0.11 | 0.33 | 22.39 | 54.40 | 6.3 |
| E3 | 1.34 | 1.35 | 6.72 | 0.4 | 0.11 | 1.01 | 4.46 | 29.92 | 9.98 |
| E4 | 1.34 | 1.35 | 4.03 | 0.4 | 0.11 | 1.01 | 7.44 | 28.71 | 7.94 |
| E5 | 2.83 | 1.42 | 5.67 | 0.08 | 0.09 | 0.50 | 5.57 | 45.05 | 12.56 |
| E6 | 1.42 | 2.83 | 5.67 | 0.08 | 0.09 | 1.99 | 11.09 | 45.05 | 26.64 |
| E7 | 2.32 | 0.46 | 4.1 | 0.24 | 0.46 | 0.20 | 2.49 | 29.65 | 5.5 |
| E8 | 4.1 | 0.46 | 2.32 | 0.24 | 0.46 | 0.11 | 4.41 | 46.64 | 4.48 |
| CE1 | 5.67 | 2.83 | 1.42 | 0.08 | 0.09 | 0.50 | 44.29 | 85.64 | 21.24 |
| CE2 | 2.83 | 5.67 | 1.42 | 0.08 | 0.09 | 2.00 | 88.73 | 85.64 | 86.18 |
| CE3 | 1.42 | 5.67 | 2.83 | 0.08 | 0.09 | 3.99 | 44.52 | 72.17 | 92.56 |

**Table 2: The composition, solid content and viscosity of the needle solutions of Comparative Examples 4 to 7 (CE4 to CE7).**

| No. | The composition of the needle solution | | | | Solid content (wt%) | Viscosity mPa.s (cP) |
|---|---|---|---|---|---|---|
| | HA (wt%) | Cross-linked HA aqueous solution 2 (wt%) | CMC (wt%) | Tween-20 (v/w%) | | |
| CE4 | 1 | 0.135 | -- | -- | 9.94 | 13.62 |
| CE5 | 1 | 0.067 | -- | -- | 9.97 | 8.76 |
| CE6 | 1 | 0.026 | -- | -- | 11.05 | 9.46 |
| CE7 | 3 | 0.15 | 1 | 0.000018 | 3.85 | 24.08 |

### Test Example 1: needle length evaluation

The anti-wrinkle microneedle patches of Examples 1 to 8 and Comparative Examples 1 to 7 were adopted for this test example. Specifically, the vertical distance from the base where the needle was formed to the needle tip was directly measured by observing with microscope. 5 needles of each group were randomly picked for the measurement, and the results were averaged to obtain the needle length of the anti-wrinkle microneedle patches of Examples 1 to 8 and Comparative Examples 1 to 7, which were listed in the following Table 3.

### Test Example 2: mechanical strength evaluation

The anti-wrinkle microneedle patches of Examples 1 to 8 and Comparative Examples 1 to 7 were adopted for this test example. Specifically, the anti-wrinkle microneedle patches of Examples 1 to 8 and Comparative Examples 1 and 7 were placed in an universal testing machine (model: 3343, purchased from INSTRON), and then the mechanical strength of the needle of each microneedle patch was measured. In this test example, a compressive test with the displacement set to 10 millimeters (mm) and the speed set to 66 mm/min was conducted, and 500 compressive stress values were received per second at the same time. The mechanical strength of the needles of the microneedle patches of Examples 1 to 8 and Comparative Examples 1 to 7 were listed in the following Table 3.

**Table 3: The needle length and the mechanical strength of the needle of the anti-wrinkle microneedle patches of Examples 1 to 8 (E1 to E8) and Comparative Examples 1 to 7 (CE1 to CE 7).**

| No. | Needle length (µm) | Mechanical strength of the needle (N/needle) |
|---|---|---|
| E1 | 297.24 | 0.205 |
| E2 | 299.44 | 0.206 |
| E3 | 290.56 | 0.190 |
| E4 | 299.62 | 0.170 |
| E5 | 299.34 | 0.161 |
| E6 | 302.44 | 0.144 |
| E7 | 303.87 | 0.163 |
| E8 | 300.75 | 0.169 |
| CE1 | 302.38 | 0.155 |
| CE2 | 299.38 | 0.161 |
| CE3 | 297.64 | 0.158 |
| CE4 | 301.48 | 0.115 |
| CE5 | 301.48 | 0.133 |
| CE6 | 300.78 | 0.121 |
| CE7 | 303.24 | 0.164 |

According to the results in Table 3, the needle length of the anti-wrinkle microneedle patches of Examples 1 to 8 and Comparative Examples 1 to 7 were all about 300 µm, which indicated that the needle length of the microneedle patch prepared by the method of the present invention had consistency, and had the advantage of stable quality. Further referring to the results of the mechanical strength of the needle, the mechanical strength of the needle of the anti-wrinkle microneedle patches of Examples 1 to 8 were all higher than 0.058 N/needle, and thus could pierce the stratum corneum without breakage. Therefore, the anti-wrinkle microneedle patches of the present invention could pierce the stratum corneum without bending or breakage, which ensured needle penetration under the skin and exerted the desired effects.

### Test Example 3: dissolution rate evaluation

Samples having the same composition with the needle of the anti-wrinkle microneedle patches of Examples 1 to 8 and Comparative Examples 1 to 7 were prepared according the aforementioned preparation method for the dissolution rate evaluation. Next, the samples of Examples 1 to 8 and Comparative Examples 1 to 7 were cut to the same size and weighed, such that those samples had the same weight. Then, after the samples were soaked in water for 5 minutes, the samples were taken out and weighed again. The weight difference between the sample before soaking and the sample after soaking relative to the weight of the sample before soaking in percentage was set as dissolution rate in water for 5 minutes. Here, the higher dissolution rate in water for 5 minutes indicated HA was dissolved and released more rapidly. The dissolution rate in water for 5 minutes of Examples 1 to 8 and Comparative Examples 1 to 7 were listed in the following Table 4.

**Table 4: The dissolution rate in water for 5 minutes of Examples 1 to 8 and Comparative Examples 1 to 7.**

| No. | Dissolution rate in water for 5 minutes (%) |
|---|---|
| E1 | 69.3 |
| E2 | 74.03 |
| E3 | 76.64 |
| E4 | 80.66 |
| E5 | 85.87 |
| E6 | 78.41 |
| E7 | 80.98 |
| E8 | 70.98 |
| CE1 | 24.35 |
| CE2 | 17.13 |
| CE3 | 16.58 |
| CE4 | 53.85 |
| CE5 | 56.25 |
| CE6 | 52.08 |
| CE7 | 37.26 |

According to the results in Table 4, the dissolution rates in water for 5 minutes of Examples 1 to 8 were all higher than 60%; wherein, the dissolution rates in water for 5 minutes of Examples 2 and 8 were higher than 70%, the dissolution rates in water for 5 minutes of Examples 3 and 6 were higher than 75%, and the dissolution rates in water for 5 minutes of Examples 4, 5 and 7 were higher than 80%. In contrast, the dissolution rates in water for 5 minutes of Comparative Examples 1 to 7 were all lower than 60%, and the difference of dissolution rate between Comparative Example 5 (56.25%, highest in Comparative Examples) and Example 1 (69.3%, lowest in Examples) was still up to 20%. Accordingly, as the anti-wrinkle microneedle patch of the present invention adopted the needle having specific composition, HA actually could rapidly dissolve and be released, thereby exerting the effects of skin moisturizing and increasing skin thickness and strength, and achieving the purpose of smoothing and reducing wrinkles.

### Test Example 4: anti-wrinkle evaluation

The anti-wrinkle microneedle patch of Example 7 was adopted for this test example. Specifically, 20 subjects (6 males; 14 females; age: 31 to 57) having wrinkles at periorbital were recruited. After the subjects were applied with the anti-wrinkle microneedle patch of Example 7, the tests for skin irritation, skin moisturizing, skin thickness, skin strength and wrinkle number were conducted for completely evaluating the effect of anti-wrinkle.

### (1) Skin irritation test

In this test, the anti-wrinkle microneedle patch of Example 7 was cut into a size of 2.5 cm² as a test group, and then was flat pasted on an inner side of a lower arm of the subject. Besides, a 0.9 wt% of sodium chloride solution (purchased from TUNGHSIN BIOSCIENCE CO., LTD.) was set as a control group, and then 0.2 ml of the sodium chloride solution was adsorbed by a sterile gauze of a size of 2.5 cm². The sterile gauze was also flat pasted on the inner side of the lower arm of the subject and fixed with a waterproof and breathable tape. The evaluation of skin irritation was achieved by observing whether the symptoms of skin irritation appeared on the area applied with the test group after the subject was continuously applied with the test group and the control group for 24 hours. According to the score standard of skin irritation denoted in ISO 10993-10:2010, score 0 indicated no irritated reaction; score 1 indicated weak positive reaction (mild erythema and/or dryness of the skin); score 2 indicated moderate positive reaction (obvious erythema and/or dryness of the skin, and may spread beyond the applied area); and score 3 indicated strong reaction (intense and diffuse erythema with edema and/or eschar formation). Then, the score of skin irritation provided by each subject was added and then divided by the number of the subject to obtain an index of skin irritation of the test group. The index of skin irritation lower than 5 was considered as safe level; the index of skin irritation between 5 and 15 was considered as acceptable level; and the index of skin irritation higher than 15 was considered as mild irritation level.

After the foresaid skin irritation test for 24 hr, the scores of skin irritation of the anti-wrinkle microneedle patch of Example 7 acquired from the 20 subjects were all 0, *i.e.,* no irritated reaction. Accordingly, the index of skin irritation of the anti-wrinkle microneedle patch of Example 7 was also 0, which represented the safe level without skin irritation.

### (2) Skin moisturizing test

In this test example, the subjects who completed the foresaid skin irritation test were further tested for the effect of skin moisturizing of the anti-wrinkle microneedle patch of Example 7. Specifically, the anti-wrinkle microneedle patch of Example 7 was set as a test group and pasted from the area below the right eye to corner of the right eye; and the needle solution of Example 7 was set as a control group and directly applied from the area below the left eye to corner of the left eye. The applying frequency of each group was 2 times per week for 4 weeks, and the period of applying those group each time was about 6 hr. At the times when those groups were not applied yet, *i.e.,* the 0 week, those groups were continuously applied for 2 weeks, and those groups were continuously applied for 4 weeks, the water content and the ability of moisturizing at the superficial skin of periorbital were measured by Corneometer^{®} (purchased from Courage+Khazaka electronic GmbH) based on bioelectricity. After applied with the anti-wrinkle microneedle patch of Example 7 and the needle solution of Example 7 for different time periods, the results of water content at the skin of periorbital were measured and listed in the following Table 5; wherein, the higher value indicated better ability of moisturizing, and the unit was represented by A.C.U (arbitrary capacitance units). In Table 5, the "difference relative to 0 week" indicated the water content of the specific week subtracted the water content of 0 week, and then divided by the water content of 0 week and presented in percentage, which represented the elevation degree of the water content.

**Table 5: The measured results of the water content at the skin of periorbital after applied with the anti-wrinkle microneedle patch of Example 7 and the needle solution of Example 7 for different time periods.**

| Time periods | Anti-wrinkle microneedle patch of Example 7 | | Needle solution of Example 7 | |
|---|---|---|---|---|
| | Water content (A.U.C) | Difference relative to 0 week (%) | Water content (A.U.C) | Difference relative to 0 week (%) |
| 0 week | 53.8 | -- | 51.8 | -- |
| 2 weeks | 55.4 | 3.3 | 52.6 | 1.8 |
| 4 weeks | 56.8 | 5.8 | 52.6 | 1.8 |

According to the results of Table 5, during the 4 weeks of applying the anti-wrinkle microneedle patch of Example 7, the measured water content at the skin of periorbital was gradually elevated; wherein, the measured water content of 2 weeks elevated by 3.3% compared to 0 week, and the measured water content of 4 weeks further elevated by 5.8% compared to 0 week. As for the results of the needle solution of Example 7, although the measured water content of 2 weeks elevated by 1.8% compared to 0 week, the measured water content of 4 weeks did not elevate compared to 0 week, and regardless of 2 weeks or 4 weeks, the effect of elevating the water content at the skin of periorbital was obviously worse than the anti-wrinkle microneedle patch of Example 7. Accordingly, further making the needle solution of Example 7 into the microneedle patch actually could provide better effect of elevating the water content at the skin of periorbital and had better ability of moisturizing.

### (3) Skin thickness and strength test

This test and the foresaid (2) skin moisturizing test were conducted at the same time. At the times when the anti-wrinkle microneedle patch of Example 7 and the needle solution of Example 7 were not applied to the subject yet, *i.e.,* the 0 week, those groups were continuously applied for 2 weeks, and those groups were continuously applied for 4 weeks, the structural change at the dermis of periorbital was evaluated by DermaLab^{®} (purchased from Cortex Technology) based on the recorded ultrasound images of the skin below the eyes and at corner of the eyes. Said DermaLab^{®} was a probe for ultrasound image of the dermis, and in the ultrasonic image, color was used to represent the intensity of ultrasonic echo; wherein, deep color indicated low intensity and white/yellow indicated high intensity. In general, the epidermis had high intensity (white and yellow); the dermis has a combination of different color such as green, red and yellow (color region comprising the structures of collagen and elastin); and the fat, muscle and vessel beneath the dermis had low intensity (deep green and black). After applied with the anti-wrinkle microneedle patch of Example 7 and the needle solution of Example 7 for different time periods, the measured results of thickness of the skin below the eyes and at corner of the eyes were listed in the following Table 6, and the higher value indicated the thicker thickness, and the unit was represented by µm; the measured results of strength of the skin below the eyes and at corner of the eyes were listed in the following Table 7, and the higher value indicated the higher strength, and the unit was represented by intensity score. In Table 6 and Table 7, the "difference relative to 0 week" indicated the result of the specific week subtracted the result of 0 week, and then divided by the result of 0 week and presented in percentage, which represented the elevation degree of the result.

**Table 6: The results of measuring the thickness of the skin below the eyes and at corner of the eyes after applied with the anti-wrinkle microneedle patch of Example 7 and the needle solution of Example 7 for different time periods.**

| Position | Time periods | Anti-wrinkle microneedle patch of Example 7 | | Needle solution of Example 7 | |
|---|---|---|---|---|---|
| | | Thickness (µm) | Difference relative to 0 week (%) | Thickness (µm) | Difference relative to 0 week (%) |
| Area below the eyes | 0 week | 888.8 | -- | 931.1 | -- |
| | 2 weeks | 1036.0 | 18.7 | 979.0 | 7.0 |
| | 4 weeks | 1121.4 | 33.1 | 1123.3 | 22.4 |
| Corner of the eyes | 0 week | 989.4 | -- | 892.8 | -- |
| | 2 weeks | 1023.3 | 11.9 | 923.1 | 5.0 |
| | 4 weeks | 1178.1 | 30.0 | 1033.8 | 17.1 |

According to the results of Table 6, during the 4 weeks of applying the anti-wrinkle microneedle patch of Example 7, both the thickness of the skin below the eyes and at corner of the eyes had a clear upward trend; wherein, for the thickness of the skin below the eyes, the result of 2 weeks elevated by 18.7% compared to 0 week, and the result of 4 weeks further elevated by 33.1%; and for the thickness of the skin at corner of the eyes, the result of 2 weeks elevated by 11.9% compared to 0 week, and the result of 4 weeks further elevated by 30.0% compared to 0 week. As for the results of the needle solution of Example 7, for the thickness of the skin below the eyes, the results of 2 weeks and 4 weeks respectively elevated by 7.0% and 22.4% compared to 0 week; and for the thickness of the skin at corner of the eyes, the results of 2 weeks and 4 weeks respectively elevated by 5.0% and 17.1% compared to 0 week. Accordingly, the effect of the needle solution of Example 7 for elevating the thickness of the skin below the eyes and at corner of the eyes was obviously worse than the anti-wrinkle microneedle patch of Example 7. That is, further making the needle solution of Example 7 into the microneedle patch actually could provide better effect of elevating the thickness of the skin of periorbital.

**Table 7: The results of measuring the strength of the skin below the eyes and at corner of the eyes after applied with the anti-wrinkle microneedle patch of Example 7 and the needle solution of Example 7 for different time periods.**

| Position | Time periods | Anti-wrinkle microneedle patch of Example 7 | | Needle solution of Example 7 | |
|---|---|---|---|---|---|
| | | Strength (intensity score) | Difference relative to 0 week (%) | Strength (intensity score) | Difference relative to 0 week (%) |
| Area below the eyes | 0 week | 27.5 | -- | 29.0 | -- |
| | 2 weeks | 30.6 | 15.5 | 28.7 | 13.0 |
| | 4 weeks | 34.8 | 34.8 | 28.3 | 12.3 |
| Corner of the eyes | 0 week | 26.1 | -- | 25.2 | -- |
| | 2 weeks | 28.3 | 10.3 | 26.5 | 10.2 |
| | 4 weeks | 30.0 | 18.0 | 27.3 | 14.8 |

According to the results of Table 7, during the 4 weeks of applying the anti-wrinkle microneedle patch of Example 7, both the strength of the skin below the eyes and at corner of the eyes had a clear upward trend; wherein, for the strength of the skin below the eyes, the result of 2 weeks elevated by 15.5% compared to 0 week, and the result of 4 weeks further elevated by 34.8%; and for the strength of the skin at corner of the eyes, the result of 2 weeks elevated by 10.3% compared to 0 week, and the result of 4 weeks further elevated by 18.0% compared to 0 week. As for the results of the needle solution of Example 7, for the strength of the skin below the eyes, the results of 2 weeks and 4 weeks respectively elevated by 13.0% and 12.3% compared to 0 week; and for the strength of the skin at corner of the eyes, the results of 2 weeks and 4 weeks respectively elevated by 10.2% and 14.8% compared to 0 week. Accordingly, the effect of the needle solution of Example 7 for elevating the strength of the skin below the eyes and at corner of the eyes was obviously worse than the anti-wrinkle microneedle patch of Example 7. That is, further making the needle solution of Example 7 into the microneedle patch actually could provide better effect of elevating the strength of the skin of periorbital.

### (4) Wrinkle reduction test

This test and the foresaid (2) skin moisturizing test were conducted at the same time. At the times when the anti-wrinkle microneedle patch of Example 7 and the needle solution of Example 7 were not applied to the subject yet, *i.e.,* the 0 week, those groups were continuously applied for 2 weeks, and those groups were continuously applied for 4 weeks, the number of wrinkle at corner of the eyes was analyzed by VISIA^{®}Evolution ver. 7.0.1 (purchased from Canfield Scientific, Inc.) based on the high-resolution (12 million pixels) image of the skin at corner of the eyes. After applied with the anti-wrinkle microneedle patch of Example 7 and the needle solution of Example 7 for different time periods, the numbers of wrinkle at corner of the eyes were listed in the following Table 8, and the lower value indicated the less number of wrinkle, and the unit was represented by count. In Table 8, the "difference relative to 0 week" indicated the number of wrinkle of the specific week subtracted the number of wrinkle of 0 week, and then divided by the number of wrinkle of 0 week and presented in percentage, which represented the reduction degree of the wrinkles.

**Table 8: The results of the number of wrinkle at corner of the eyes after applied with the anti-wrinkle microneedle patch of Example 7 and the needle solution of Example 7 for different time periods.**

| Time periods | Anti-wrinkle microneedle patch of Example 7 | | Needle solution of Example 7 | |
|---|---|---|---|---|
| | Number of wrinkle (count) | Difference relative to 0 week (%) | Number of wrinkle (count) | Difference relative to 0 week (%) |
| 0 week | 98.5 | -- | 97.2 | -- |
| 2 weeks | 95.0 | -1.4 | 95.2 | -1.0 |
| 4 weeks | 92.2 | -4.4 | 94.0 | -2.0 |

According to the results of Table 8, during the 4 weeks of applying the anti-wrinkle microneedle patch of Example 7, the measured number of wrinkle at corner of the eyes was gradually decreased; wherein, the measured number of wrinkle of 2 weeks decreased by 1.4% compared to 0 week, and the measured number of wrinkle of 4 weeks further decreased by 4.4% compared to 0 week. As for the results of the needle solution of Example 7, the numbers of wrinkle of 2 weeks and 4 weeks respectively decreased by 1.0% and 2.0% compared to 0 week, which clearly indicated that regardless of being applied with the needle solution of Example 7 for 2 weeks or 4 weeks, the effect of reducing the number of wrinkle at corner of the eyes was obviously worse than the anti-wrinkle microneedle patch of Example 7. Accordingly, further making the needle solution of Example 7 into the microneedle patch actually could provide better effect of smoothing wrinkles and had better ability of wrinkle reduction.

### Test Example 5: comparison between anti-wrinkle microneedle patch and known anti-wrinkle materials

This test example adopted the effects of elevating the skin moisturizing of periorbital, elevating the thickness and strength of the skin below the eyes and reducing the number of wrinkle at corner of eyes of the anti-wrinkle microneedle patch of Example 7 denoted in the Test Example 4, and further compared to a commercial anti-wrinkle product: La Mer^{®} cream (hereinafter referred to as control group 1) and a known anti-wrinkle substance: retinyl retinoate (hereinafter referred to as control group 2). Specifically, the control group 1 was applied once per day for 4 weeks, so as to show the influences on the skin moisturizing and the number of wrinkles compared to 0 week; and the control group 2 was applied twice per day for 12 weeks to show the influences on the thickness and strength compared to 0 week. For comparing the differences in effect among the different groups, the measured results of the skin moisturizing, the thickness and strength of the skin and the number of wrinkle of the anti-wrinkle microneedle patch of Example 7, the control group 1 and the control group 2 were listed in the following Table 9.

**Table 9: The test conditions and the measured results of the skin moisturizing, the thickness and strength of the skin and the number of wrinkle of the anti-wrinkle microneedle patch of Example 7 (E7), the control group 1 (C1) and the control group 2 (C2).**

| No. | Test conditions | | Elevation of the skin moisturizing (relative to 0 week) | Elevation of the thickness of the skin (relative to 0 week) | Elevation of the strength of the skin (relative to 0 week) | Reduction of the number of wrinkles (relative to 0 week) |
|---|---|---|---|---|---|---|
| | Applying frequency | Time period | | | | |
| E7 | twice /week | 4 weeks | 5.8% | 33.1% | 34.8% | 4.4% |
| C1 | once /day | 4 weeks | 6% | -- | -- | 3% |
| C2 | Twice /day | 12 weeks | -- | 9.3% | 25.88% | -- |

According to the results of Table 9, in comparison with the control group 1, the anti-wrinkle microneedle patch of Example 7 had comparable effect of elevating the skin moisturizing, and had better effect of reducing the number of wrinkles simultaneously. Especially, in the situation that the applying frequency of the anti-wrinkle microneedle patch of Example 7 was obviously lower than that of the control group 1, the anti-wrinkle microneedle patch of Example 7 should substantially have superior effects of elevating the skin moisturizing and reducing the number of wrinkles compared to the control group 1. Further compared to the control group 2, the anti-wrinkle microneedle patch of Example 7 had better effect of elevating the thickness and strength of the skin simultaneously. Besides, the applying frequency of the anti-wrinkle microneedle patch of Example 7 was obviously lower than that of the control group 2. That is to say, the anti-wrinkle microneedle patch of Example 7 should also substantially have superior effects of elevating the thickness and strength of the skin compared to the control group 2. Accordingly, in comparison with the known anti-wrinkle materials, the anti-wrinkle microneedle patch of the present invention actually had better effects of elevating the skin moisturizing, elevating the thickness and strength of the skin and reducing the number of wrinkles.

In conclusion, by controlling the composition and the ratio of each ingredient of the anti-wrinkle composition of the present invention, and further taking the anti-wrinkle composition as the material of the needles of the microneedle patch, the prepared anti-wrinkle microneedle patch not only has good mechanical strength contributing to applying the microneedle patch, but also makes HA rapidly dissolve and released, so as to exert the effects of skin moisturizing and increasing skin thickness and strength, and achieve the purpose of smoothing and reducing wrinkles, thereby having great commercial value.

## Claims

1. An anti-wrinkle composition, **characterized by** comprising a low-molecular hyaluronic acid, a modified hyaluronic acid and a cross-linked hyaluronic acid, and a weight ratio of the modified hyaluronic acid relative to the low-molecular hyaluronic acid being more than or equal to 0.1 and less than or equal to 4, and a weight ratio of the modified hyaluronic acid relative to the cross-linked hyaluronic acid being less than 30:
wherein an average molecular weight of the low-molecular hyaluronic acid is more than or equal to 1 kDa and less than or equal to 10 kDa, and the modified hyaluronic acid is hyaluronic acid modified with histidine.

2. The anti-wrinkle composition as claimed in claim 1, **characterized in that** the cross-linked hyaluronic acid is prepared by subjecting hyaluronic acid to a cross-linking agent for cross-linking reaction.

3. The anti-wrinkle composition as claimed in claim 1 or 2, **characterized in that** the anti-wrinkle composition further comprises p-hydroxyacetophenone, pentylene glycol, hexanediol, caprylyl glycol, phenoxyethanol, benzyl alcohol, salicylic acid, benzoic acid, sorbic acid, potassium sorbate, propionic acid, dehydroacetic acid, chlorophenesin, behentrimonium chloride, benzalkonium chloride, benzethonium chloride, butyl benzoate, dimethyl oxazolidine, bromochlorophene, dichlorobenzyl alcohol, ethyl lauroyl arginate HCl, 7-ethylbicyclooxazolidine, formic acid, glutaral, hexamidine, sodium sulfite, iodopropynyl butylcarbamate, methylisothiazolinone, butylparaben, methylparaben, phenoxyisopropanol, phenylmercuric acetate, triclocarban, undecylenic acid, zinc pyrithione, 5-bromo-5-nitro-1,3-dioxane, benzylhemiformal, 1,3-dimethylol-5,5-dimethylhydantoin, diazolidinyl urea, imidazolidinyl urea, methenamine, quaternium-15, sodium hydroxymethylglycinate, Leuconostoc/Radish Root Ferment Filtrate, Lactobacillus ferment, Populus Tremuloides Bark Extract, Black Currant Fruit Extract or any combination thereof.

4. An anti-wrinkle microneedle patch, **characterized by** comprising a base and multiple needles; the base having an upper surface and the multiple needles formed on the upper surface; wherein, a material of each needle comprises the anti-wrinkle composition as claimed in any one of claims 1 to 3.

5. A preparation method of the anti-wrinkle microneedle patch as claimed in claim 4, **characterized by** comprising the following steps:
step (a): providing a master mold having a datum plane and multiple holes, and the multiple holes formed by recessing from the datum plane;
step (b): filling the multiple holes with a needle solution to make a surface of the needle solution level with the datum plane; wherein the needle solution comprises a low-molecular hyaluronic acid, a modified hyaluronic acid and a cross-linked hyaluronic acid, and a weight ratio of the modified hyaluronic acid relative to the low-molecular hyaluronic acid is more than or equal to 0.1 and less than or equal to 4, and a weight ratio of the modified hyaluronic acid relative to the cross-linked hyaluronic acid is less than 30; wherein an average molecular weight of the low-molecular hyaluronic acid is more than or equal to 1 kDa and less than or equal to 10 kDa, and the modified hyaluronic acid is hyaluronic acid modified with histidine;
step (c): drying the needle solution to form multiple needles, wherein a surface of each needle is lower than the datum plane;
step (d): filling the multiple holes with a base solution, and the base solution covering the surface of the needles and the datum plane;
step (e): drying the base solution to form a base, such that the base is adhered to the multiple needles; and
step (f): demolding the needles and the base that are adhered to each other from the master mold to obtain the anti-wrinkle microneedle patch.

6. The preparation method as claimed in claim 5, **characterized in that** a sum of a weight of the low-molecular hyaluronic acid, a weight of the modified hyaluronic acid, and a weight of the cross-linked hyaluronic acid in 1000 g of the needle solution is more than or equal to 20 g and less than or equal to 70 g.

7. The preparation method as claimed in claim 5 or 6, **characterized in that** a viscosity of the needle solution is more than or equal to 2 mPa.s (cP) and less than or equal to 50 mPa.s (cP).

## Patentansprüche

1. Anti-Falten-Zusammensetzung, **dadurch gekennzeichnet, dass** sie eine niedermolekulare Hyaluronsäure, eine modifizierte Hyaluronsäure und eine vernetzte Hyaluronsäure umfasst, und ein Gewichtsverhältnis der modifizierten Hyaluronsäure relativ zu der niedermolekularen Hyaluronsäure mehr als oder gleich 0,1 und weniger als oder gleich 4 beträgt und ein Gewichtsverhältnis der modifizierten Hyaluronsäure relativ zu der vernetzten Hyaluronsäure weniger als 30 beträgt:
wobei ein durchschnittliches Molekulargewicht der niedermolekularen Hyaluronsäure mehr als oder gleich 1 kDa und weniger als oder gleich 10 kDa beträgt und die modifizierte Hyaluronsäure mit Histidin modifizierte Hyaluronsäure ist.

2. Anti-Falten-Zusammensetzung gemäß Anspruch 1, **dadurch gekennzeichnet, dass** die vernetzte Hyaluronsäure hergestellt wird, indem Hyaluronsäure einem Vernetzungsmittel zur Vernetzungsreaktion ausgesetzt wird.

3. Anti-Falten-Zusammensetzung gemäß Anspruch 1 oder 2, **dadurch gekennzeichnet, dass** die Anti-Falten-Zusammensetzung ferner p-Hydroxyacetophenon, Pentylenglykol, Hexandiol, Caprylylglykol, Phenoxyethanol, Benzylalkohol, Salicylsäure, Benzoesäure, Sorbinsäure, Kaliumsorbat Propionsäure, Dehydroessigsäure, Chlorphenesin, Behentrimoniumchlorid, Benzalkoniumchlorid, Benzethoniumchlorid, Butylbenzoat, Dimethyloxazolidin, Bromchlorophen, Dichlorbenzylalkohol, Ethyllauroylarginat HCl, 7-Ethylbicyclooxazolidin, Ameisensäure, Glutaral, Hexamidin, Natriumsulfit, lodopropynylbutylcarbamat, Methylisothiazolinon, Butylparaben, Methylparaben, Phenoxyisopropanol, Phenylmercurylacetat, Triclocarban, Undecylensäure, Zinkpyrithion, 5-Brom-5-nitro-1,3-dioxan, Benzylhemiformal, 1,3-Dimethylol-5, 5-Dimethylhydantoin, Diazolidinylharnstoff, Imidazolidinylharnstoff, Methenamin, Quaternium-15, Natriumhydroxymethylglycinat, Leuconostoc/Radieschenwurzelferment-Filtrat, Lactobacillus ferment, Populus Tremuloides Rindenextrakt, Schwarzer Johannisbeerfruchtextrakt oder eine beliebige Kombination davon.

4. Anti-Falten-Mikronadelpflaster, **dadurch gekennzeichnet, dass** es eine Basis und mehrere Nadeln umfasst, wobei die Basis eine obere Fläche und die mehreren Nadeln auf der oberen Fläche ausgebildet sind, wobei ein Material jeder Nadel die Anti-Falten-Zusammensetzung gemäß einem der Ansprüche 1 bis 3 umfasst.

5. Herstellungsverfahren für das Antifalten-Mikronadelpflaster gemäß Anspruch 4, **dadurch gekennzeichnet, dass** es die folgenden Schritte umfasst:
Schritt (a): Bereitstellen einer Mutterform mit einer Bezugsebene und mehreren Löchern, wobei die mehreren Löcher durch Aussparung von der Bezugsebene gebildet werden;
Schritt (b): Füllen der mehreren Löcher mit einer Nadellösung, um eine Oberfläche der Nadellösung mit der Bezugsebene bündig zu machen; wobei die Nadellösung eine niedermolekulare Hyaluronsäure, eine modifizierte Hyaluronsäure und eine vernetzte Hyaluronsäure umfasst und das Gewichtsverhältnis der modifizierten Hyaluronsäure zu der niedermolekularen Hyaluronsäure mehr als oder gleich 0, 1 ist und weniger als oder gleich 4 ist, und ein Gewichtsverhältnis der modifizierten Hyaluronsäure relativ zu der vernetzten Hyaluronsäure weniger als 30 ist; wobei ein durchschnittliches Molekulargewicht der niedermolekularen Hyaluronsäure mehr als oder gleich 1 kDa und weniger als oder gleich 10 kDa ist, und die modifizierte Hyaluronsäure mit Histidin modifizierte Hyaluronsäure ist;
Schritt (c): Trocknen der Nadellösung, um mehrere Nadeln zu bilden, wobei eine Oberfläche jeder Nadel niedriger als die Bezugsebene ist;
Schritt (d): Füllen der mehreren Löcher mit einer Basislösung, wobei die Basislösung die Oberfläche der Nadeln und die Bezugsebene bedeckt;
Schritt (e): Trocknen der Basislösung, um eine Basis zu bilden, so dass die Basis an den mehreren Nadeln anhaftet; und
Schritt (f): Entformen der Nadeln und der Basis, die aneinander haften, aus der Urform, um das Anti-Falten-Mikronadelpflaster zu erhalten.

6. Herstellungsverfahren gemäß Anspruch 5, **dadurch gekennzeichnet, dass** eine Summe des Gewichts der niedermolekularen Hyaluronsäure, des Gewichts der modifizierten Hyaluronsäure und des Gewichts der vernetzten Hyaluronsäure in 1000 g der Nadellösung mehr als oder gleich 20 g und weniger als oder gleich 70 g beträgt.

7. Herstellungsverfahren gemäß Anspruch 5 oder 6, **dadurch gekennzeichnet, dass** die Viskosität der Nadellösung mehr als oder gleich 2 mPa.s (cP) und weniger als oder gleich 50 mPa.s (cP) beträgt.

## Revendications

1. Composition anti-rides, **caractérisée en ce qu'**elle comprend un acide hyaluronique de bas poids moléculaire, un acide hyaluronique modifié et un acide hyaluronique réticulé, et un rapport pondéral de l'acide hyaluronique modifié par rapport à l'acide hyaluronique de bas poids moléculaire qui est supérieur ou égal à 0,1 et inférieur ou égal à 4, et un rapport pondéral de l'acide hyaluronique modifié par rapport à l'acide hyaluronique réticulé qui est inférieur à 30 ;
dans laquelle un poids moléculaire moyen de l'acide hyaluronique de bas poids moléculaire est supérieur ou égal à 1 kDa et inférieur ou égal à 10 kDa, et l'acide hyaluronique modifié est un acide hyaluronique modifié par de l'histidine.

2. Composition anti-rides selon la revendication 1, **caractérisée en ce que** l'acide hyaluronique réticulé est préparé en soumettant l'acide hyaluronique à un agent de réticulation pour une réaction de réticulation.

3. Composition anti-rides selon la revendication 1 ou 2, **caractérisée en ce que** la composition anti-rides comprend en outre de la *p*-hydroxyacétophénone, du pentylène glycol, de l'hexanediol, du caprylyl-glycol, du phénoxyéthanol, de l'alcool benzylique, de l'acide salicylique, de l'acide benzoïque, de l'acide sorbique, du sorbate de potassium, de l'acide propionique, de l'acide déhydroacétique, de la chlorophénésine, du chlorure de béhentrimonium, du chlorure de benzalkonium, du chlorure de benzéthonium, du benzoate de butyle, de la diméthyloxazolidine, du bromochlorophène, de l'alcool dichlorobenzylique, de l' arginate d'éthyl-lauroyle HCl, de la 7-éthylbicyclooxazolidine, de l'acide formique, du glutaral, de l'hexamidine, du sulfite de sodium, du butylcarbamate d'iodopropynyle, de la méthylisothiazolinone, du butylparabène, du méthylparabène, du phénoxyisopropanol, de l'acétate phénylmercurique, du triclocarban, de l'acide undécylénique, de la pyrithione de zinc, du 5-bromo-5-nitro-1,3-dioxane, du benzylhémiformal, de la 1,3-diméthylol-5,5-diméthylhydantoïne, de la diazolidinylurée, de l'imidazolidinylurée, de la méthénamine, du quaternium-15, de l'hydroxyméthylglycinate de sodium, un filtrat de ferment de leuconostoc/racine de radis, un ferment de Lactobacillus, un extrait d'écorce de Populus Tremuloides, un extrait de fruit de cassis ou quelconque combinaison de ceux-ci.

4. Patch à micro-aiguilles anti-rides, **caractérisé en ce qu'**il comprend une base et de multiples aiguilles ; la base ayant une surface supérieure et les multiples aiguilles étant formées sur la surface supérieure ; dans lequel un matériau de chaque aiguille comprend la composition anti-rides selon l'une quelconque des revendications 1 à 3.

5. Procédé de préparation du patch à micro-aiguilles anti-rides selon la revendication 4, **caractérisé en ce qu'**il comprend les étapes suivantes :
étape (a) : fourniture d'un moule maître ayant un plan de référence et de multiples trous, et les multiples trous étant formés par évidement à partir du plan de référence ;
étape (b) : remplissage des multiples trous avec une solution d'aiguille pour faire une surface de la solution d'aiguille au niveau du plan de référence ;
dans lequel la solution d'aiguille comprend un acide hyaluronique de bas poids moléculaire, un acide hyaluronique modifié et un acide hyaluronique réticulé, et un rapport pondéral de l'acide hyaluronique modifié par rapport à l'acide hyaluronique de bas poids moléculaire qui est supérieur ou égal à 0,1 et inférieur ou égal à 4, et un rapport pondéral de l'acide hyaluronique modifié par rapport à l'acide hyaluronique réticulé qui est inférieur à 30 ; dans lequel un poids moléculaire moyen de l'acide hyaluronique de bas poids moléculaire est supérieur ou égal à 1 kDa et inférieur ou égal à 10 kDa, et l'acide hyaluronique modifié est un acide hyaluronique modifié par de l'histidine ;
étape (c) : séchage de la solution d'aiguille pour former de multiples aiguilles, la surface de chaque aiguille étant inférieure au plan de référence ;
étape (d) : remplissage des multiples trous avec une solution de base, et la solution de base recouvrant la surface des aiguilles et le plan de référence ;
étape (e) : séchage de la solution de base pour former une base, de telle sorte que la base adhère aux multiples aiguilles ; et
étape (f) : démoulage des aiguilles et de la base qui adhèrent ensemble du moule maître pour obtenir le patch à micro-aiguilles anti-rides.

6. Procédé de préparation selon la revendication 5, **caractérisé en ce que** la somme d'un poids de l'acide hyaluronique de faible poids moléculaire, d'un poids de l'acide hyaluronique modifié et d'un poids de l'acide hyaluronique réticulé dans 1000 g de la solution d'aiguille est supérieure ou égale à 20 g et inférieure ou égale à 70 g.

7. Procédé de préparation selon la revendication 5 ou 6, **caractérisé en ce qu'**une viscosité de la solution d'aiguille est supérieure ou égale à 2 mPa.s (cP) et inférieure ou égale à 50 mPa.s (cP).
